(19) Europäisches Patentamt
European Patent Office
Office européen des brevets

(11) Publication number: 0 244 147
A2

(12) # EUROPEAN PATENT APPLICATION

(21) Application number: 87303499.5

(22) Date of filing: 22.04.87

(51) Int. Cl.4: C 12 P 21/02
// C12N15/00

(30) Priority: 28.04.86 US 856912

(43) Date of publication of application:
04.11.87 Bulletin 87/45

(84) Designated Contracting States:
CH DE FR GB IT LI NL

(71) Applicant: MERCK & CO. INC.
126, East Lincoln Avenue P.O. Box 2000
Rahway New Jersey 07065-0900 (US)

(72) Inventor: Sha, Amanda M.
18 Cool Valley Road
Malvern Pennsylvania 19355 (US)

Lehman, E. Dale
104 Crestwood Drive
Landsale Pennsylvania 19446 (US)

(74) Representative: Hesketh, Alan, Dr.
European Patent Department Merck & Co., Inc. Terlings
Park Eastwick Road
Harlow Essex, CM20 2QR (GB)

(54) **Purification process for hybrid proteins.**

(57) A hybrid protein containing a β-galactosidase moiety linked to a desired protein through a renin cleavage site is adsorbed to an affinity matrix under conditions in which no or minimal nonspecific adsorption occurs. The matrix-hybrid protein complex is then washed with buffer to remove loosely bound or associated proteins and resuspended in incubation buffer and renin is added to cleave the hybrid protein from the complex. The desired protein is released to the supernatant liquid while the β-galactosidase moiety remains bound to the affinity matrix. Purification of the desired protein is accomplished by anion exchange chromatography.

EP 0 244 147 A2

## Description

# PURIFICATION PROCESS FOR HYBRID PROTEINS

## BACKGROUND OF THE INVENTION

The expression of a desired protein as a hybrid protein from a transformed host using recombinant DNA techniques is known in the art. In the past, the desired protein was isolated by subjecting a crude cell extract to ammonium sulfate precipitation, ion exchange chromatography and multiple separations by gel filtration chromatography. These processes do not preclude the possibilities of protein aggregation and degradation. The yield is consistently low and the product contains significant impurities. In addition, these processes are time consuming and ordinarily take from about 1 week to about 10 days or more.

## SUMMARY OF THE INVENTION

A hybrid protein containing a β-galactosidase moiety linked to a desired peptide through a renin cleavage site is adsorbed to an affinity matrix under conditions in which no or minimal nonspecific adsorption occurs. The matrix-hybrid protein complex is then washed with buffer to remove loosely bound or associated proteins. The matrix is then resuspended in incubation buffer and renin is added to cleave the hybrid protein. The desired peptide is released to the supernatant liquid while the β-galactosidase moiety remains bound to the affinity matrix. Purification of the desired protein is accomplished by anion exchange chromatography.

## OBJECTS OF THE INVENTION

It is, accordingly, an object of the present invention to provide an improved process for isolating a desired protein from a hybrid protein. Another object is to provide an improved process for isolating a desired protein in a shorter period time. Still another object is to provide a process which eliminates the possibilites of protein aggregation and degradation. A further object is to provide a process which provides higher yield and superior purity of the desired protein. These and other objects of the present invention will be apparent from the following description.

## DETAILED DESCRIPTION

The present invention relates to a rapid and efficient process for the isolation of a desired protein from a hybrid protein. The term desired protein as used herein, includes not only native and modified proteins but also polypeptides and peptides derived from native or modified proteins. The hybrid protein contains the desired protein sequentially attached to an enzyme cleavage site which in turn is attached to a stably expressed protein. The desired protein is produced as a fusion or hybrid protein from a transformed host using recombinant DNA techniques known to those skilled in the art. The hybrid protein is expressed from a vector wherein the DNA coding for the desired protein and that coding for the stably expressed protein are linked by a segment of DNA coding for an enzymatic cleavage site recognized by the enzyme renin. Consequently, the expressed hybrid protein can be cleaved by renin to separate the desired protein from the stably expressed protein. The construction of hybrid proteins containing a site recognized by the enzyme renin is disclosed in U.S. patent Application Serial No. 614,085, filed 24 May 1985, the disclosure of which is hereby incorporated by reference. Preferably, the non-desired or stably expressed portion of the hybrid protein consists of β-galactosidase attached to the desired protein through a peptide segment capable of being cleaved by the enzyme renin.

While the following description and examples illustrate the present invention with respect to a particular fusion protein containing an immunogenic fragment from the Epstein-Barr virus (EBV), it is to be understood that the present invention is applicable to any protein of interest expressed from a transformed host as a fusion protein. Examples of other proteins include viral antigens, bacterial antigens, chylamydial antigens, rickettsial antigens, fungal antigens, protozoal antigens, peptide and polypeptide hormones, growth factors, immune regulatory proteins and peptides, enzymes, oncogenic proteins, and other biologically active peptides and proteins.

According to the present invention a sample of host cells containing the hybrid protein is suspended in a buffer, preferably TRIS saline buffer about pH 7.6, containing about 200 mM TRIS, about 250 mM NaCl, about 10 mM carbonate, about 10 mM β-mercaptoethanol, about 5% glycerol, and about 0.1M phenyl-methylsulfonylflouride in isopropanol, to a concentration of about 5% to about 35%. Disruption of the cells is accomplished by either chemical or physical means, with physical disruption preferred. The supernatant fluid containing the hybrid protein is collected by centrifugation. The protein may be treated with streptomycin sulfate, about 2.5% to about 10% weight per volume to precipitate nucleic acids. The hybrid protein is collected from the supernatant liquid following centrifugation and dialyzed against an acceptable buffer prior to matrix attachment and renin cleavage. An acceptable buffer must have the appropriate pH and salt concentration for the attachment of the hybrid protein to the matrix and for renin cleavage. An acceptable buffer for the attachment and cleavage of the Epstein-Barr virus (EBV) as used in the following examples consists of about 40 mM TRIS, about 225 mM NaCl, about 10 mM $MgCl_2$, about 10mM β-mercaptoethanol and about 5 mM ethylenediamine-tetraacetic acid (buffer A) at a pH of about 7.4. The dialysis is continued for about 3 to about 24 hours at about 4° C. The hybrid protein is clarified by centrifugation and added to the affinity matrix in either a batch process or column separation techique. The affinity matrix is made up of cross linked agarose gel (AGR) linked to a p-aminophenyl-β-D-thiogalactoside (TPEG) a ligand specific for a β-galactosidase, according to the procedures of Ullmann, Gene. 29:27-31 (1984). The TPEG is attached to the agarose by the following linking structure:

$$AGR-O-\overset{NH_2^{+}}{\overset{\|}{C}}-NH-(CH_2)_5-\overset{O}{\overset{\|}{C}}-NH-TPEG$$

which is preferred for the purification of EBV immunogen containing hybrid protein.

Purification of the EBV immunogen is accomplished by the addition of about 1mg to about 20 mg hybrid protein to about 2.5 ml of setTled gel. The hybrid protein is allowed to absorb to the TPEG and linking structure for about 120 minutes, washed with buffer A to remove loosely-bound or associated proteins and resuspended in the same buffer. The hybrid protein attaches to the TPEG by the β-galacto sidase moiety while the EBV portion associateS with the positively charged linking structure. Renin is added at concentration of about 0.5 ml per about 2.5 ml of settled gel with a specific activity of about 425 units (u) per ml per 5 mg and incubated for about 7 to about 24 hours at about 37°C. The desireD protein is released into the buffer and is isolated from the supernatant fluid following centrifugation. The desired protein which has been released from the hybrid protein, may be used, without further purification, as an antigen in various diagnostic assays to determine the preserve or amount of EBV antibody in human body fluids. The desired protein is further purified by separating the renin from the desired protein by anion exchange high performance liquid chromatography (HPLC) for use as a vaccine or antigen for diagnostic assays.

The solid matrix from the above centrifugation contains the attached β-galactosidase. The β-galactosidase is removed from the matrix by elution with 0.1M Borate buffer, pH 10. The matrix is washed with buffer A and reused.

The desired protein is also purified by an affinity column technique using the same matrix. The matrix is prepared as described above and the column prepared according to the method of Ullmann, Gene. 29:37-31 (1984). The matrix packed column is washed with buffer A and a washed and clarified sample of hybrid protein is applied to the column. The hybrid protein concentration applied is similar to the batch method. After attachment has taken place the column is washed with buffer A until the effluent is devoid of detectable protein and β-galactosidase activity. Fresh buffer A containing renin is recycled through the column at about 37°C for about 7 to about 24 hours. The desired protein is isolated from the column effluent. The renin is separated from the desired protein by HPLC. The column is regenerated by treatment with 0.1M borate buffer, pH 10 to remove β-galactosidase and repeated washings with buffer A.

## EXAMPLE 1

### Isolation of Hybrid fusion Protein

All work was done at 4°C unless otherwise stated. A bacterial cell paste was prepared by mixing 50 grams of *Escherichia coli* $III_{57}$ containing the cloned hybrid protein Epstein Barr Virus-membrane antigen (EBV-MA) linked to a renin cleavage site which in turn is linked to β-galactosidase with sufficient quantity of breaking buffer (200 mM TRIS, 250 mM NaCl, 10 mM magnesium acetate, 10 mM β-mercaptoethanol and 5% glycerol) to result in a 10% weight per volume concentration. Immediately before cell rupture phenylmethylsulfonyl flouride was added to a final concentration of 2mM to inhibit serine proteases. The bacterial cells were ruptured by a single pass of homogenous suspension of the bacteria through a Stansted pressure cell at 80 psi, 12 psi back pressure.

The disrupted cell suspension was clarified by centrifugation at 10,000 x g for 60 minutes. The supernatant liquid (500 ml) was combined with 55 ml of 50% streptomycin sulfate (wt./wt.) and continuously mixed for 30 minutes to precipitate nucleic acids. The protein rich supernatant fluid was collected by centrifugation at 10,000 x g for 60 minutes

This protein rich, clarified cell extract was dialyzed against the cleavage buffer which consists of 225 mM NaCl, 40 mM TRIS, 10 mM $MgCl_2$, 10 mM β-mercaptoethanol and 5 mM ethylenediamine tetraacetic acid, (Buffer A), pH 7.4. The protein solution (500 ml) was placed in Spectrapor membrane tubing, molecular weight cut off 12,000-14,000, and dialyzed againes 30 volumes of Buffer A for 3 hours at 4°C with constant stirring.

## EXAMPLE 2

### Purification of Epstein Barr Virus-Membrane Antigen

The product from Example 1 was adjusted to a concentration 5 mg protein per ml following Lowry analysis. The β-galactosidase affinity matrix was prepared by linking p-amino-phenyl-β-D-thiogalactoside (TPEG) a β-galactosidase ligand to activated CH-Sepharose 4B gel (Pharmacia). A 1 mM solution of TPEG in 10 ml of water is mixed with 100 ml of the Sepharose beads and coupled by the addition of 7.5 mM N-acetyl-N′- (3-dimethylaminopropyl) carbodiimide in 8 ml water at pH 4.7. The reaction was allowed to continue for 24 hours at room temperature, after which the matrix was thoroughly washed with distilled water. Binding of the EBV-MA linked hybrid protein to the matrix was accomplished by adding 5 mg hybrid protein to 2.5 ml of settled matrix. The hybrid protein binds to the matrix via the β-galactosidase-TPEG coupling site while the EBV antigen associates with the positively charged linking structure. All non-bound or loosely bound hybrid protein is removed by continuous washing with buffer A until the wash is devoid of β-galactosidase activity as determined by a standard enzyme assay.

Purified renin with an activity of 425u per ml was used to cleave the EBV-MA from the β-galactosidase. The renin was added at a concentration of 0.5

ml per 2.5 ml of matrix containing 5 mg of hybrid protein. Renin cleavage was allowed to proceed overnight at 37°C in a water bath shaker. The EBV-MA was released into the supernatant fluid while the β-galactosidase remained firmly bound to the affinity matrix. The EBV-MA was separated from the renin by anion exchange high performance liquid chromatography using a Mono-Q (HR 5/5) column (Pharmacia) which binds the renin and other impurities. The EBV-MA renin mixture is loaded on the column and eluted with a NaCl gradient, 0 to 0.5M NaCl in 20 mM TRIS at a pH of 7.7. The purity of the EBV-MA was determined by polyacrylamide gel electrophoresis (page) (Laemmili, Nature 227:680, 1971), silver staining the gels (Morrissey, Anal. Biochem. 117:307-310, 1981) and immuno blotting the gels (Burnette, Anal. Biochem. 112:195-203, 1981) using anti-EBV hyperimmune human serum.

The disrupted clarified cell extract contained 25 u/mg β-galactosidase activity and constituted 100% of the recoverable protein. Attachment to the affinity matrix increased the specific activity to 1125 u/ml which constituted 1.8% of the recoverable protein. Indeed, approximately 80% of the hybrid protein will attach to the matrix and can be cleaned resulting in significant increase in yield. The EBV-MA following HPLC separation consists of a single band following PAGE and silver staining. The single band specifically reacts with anti-EBV hyperimmune human serum.

The affinity matrix can be reclaimed by treating with borate buffer, 100 mM sodium borate, 10 mM β-mercaptoethanol, pH 10. This releases the β-galactosidase and following a minimum of 2 bed volume washes the matrix can be reused.

## Claims

1. A method of purifying hybrid proteins containing a β-galactosidase moiety linked to a desired protein through a renin cleavage site comprising:
   a. attachment of the hybrid protein by the β-galactosidase moiety to an affinity matrix; and
   b. release of the desired protein from the hybrid protein by renin cleavage of the cleavage site.

2. A method according to Claim 1, step a, wherein the affinity matrix is p-amino-phenyl-β-D-thio-galactoside attached to agarose by a linking structure.

3. A method according to Claim 2 wherein the linking structure contains 7 carbon atoms.

4. A method according to Claim 2 wherein the linking structure has a positive charge.

5. A method according to Claim 2 wherein the linking structure is a compound of formula:

$$A\text{-}O\text{-}\overset{NH_2^+}{C}\text{-}NH\text{-}(CH_2)_5\text{-}\overset{O}{C}\text{-}NH\text{-}T$$

in which A represents agarose and T represents p-amino-phenyl-β-D-thio-galactoside.

6. A method according to Claim 2 wherein the agarose is cross linked agarose gel.

7. A method according to Claim 1 wherein the desired protein is separated from the renin by anion exchange chromatography and an acceptable eluant.

8. A method according to Claim 7, where the anion exchanger is trimethylammonium methyl.

9. A method according to Claim 7 wherein the desired protein is eluted with a salt gradient.

10. A method according to Claim 9 wherein the salt is sodium chloride,.

11. A method according to Claim 7 wherein the desired peptide is Epstein Barr Virus-Membrane antigen.

12. A composition comprising a desired peptide attached to β-galactosidase by a renin cleavage site bound to a ligand, p-aminophenyl-β-D-thio-galactoside which in turn is attached to agarose by a 7 carbon positively charged linking structrure.

13. A composition comprising the desired peptide of Claim 11 attached to β-galactosidase by a renin cleavage site bound to the ligand, p-amino-phenyl-β-D-thio-galactoside which in turn is attached to agarose by a 7 carbon positively charged linking structure.